# EUROPEAN PATENT APPLICATION

(11) **EP 2 698 436 A1**
(43) Date of publication of application: **19.02.2014**
(21) Application number: 12180459.5
(22) Date of filing: 14.08.2012
(51) Int. Cl.: C12Q 1/68

(54) **Colorectal cancer markers**

(71) Applicant: Max-Planck-Gesellschaft zur Förderung der Wissenschaften e.V., 80539 München (DE)
(72) Inventor: Schweiger, Michael-Ruth, 14167, Berlin (DE); Grimm, Christina, 10785, Berlin (DE); Herwig, Ralf, 14476, Potsdam (DE); Lehrach, Hans, 14129 Berlin (DE)
(74) Representative: Fanelli Haag Kilger PLLC

(57) **Abstract**

The invention relates to the identification and selection of novel genomic regions (biomarker) and the identification and selection of novel genomic region combinations which are hypermethylated in subjects with colorectal cancer compared to subjects without colorectal cancer. Nucleic acids which selectively hybridize to the genomic regions and products thereof are also encompassed within the scope of the invention as are compositions and kits containing said nucleic acids and nucleic acids for use in diagnosing prostate cancer. Further encompassed by the invention is the use of nucleic acids which selectively hybridize to one of the genomic regions or products thereof to monitor disease progression or regression in a patient and the efficacy of therapeutic regimens.

## Description

### FIELD OF THE INVENTION

The present invention is in the field of biology and chemistry. In particular, the invention is in the field of molecular biology. More particular, the invention relates to the analysis of the methylation status of genomic regions. Most particularly, the invention is in the field of diagnosing colorectal cancer.

### BACKGROUND

Colorectal cancer (CRC) is the third most common cancer in males and the second in females, with over 1.2 million new cancer cases and 608,700 deaths estimated for 2008. Colorectal cancer, commonly known as bowel cancer, is a cancer from uncontrolled cell growth in the colon or rectum (parts of the large intestine), or in the appendix. Symptoms typically include rectal bleeding and anemia which are sometimes associated with weight loss and changes in bowel habits.

Most colorectal cancers occur due to lifestyle and increasing age, a genetic predisposition is known for the HNPCC (hereditary non-polyposis colorectal cancer) subgroup. It typically starts in the lining of the bowel and, if left untreated, can grow into the muscle layers underneath, and then through the bowel wall. Regular endoscopic control screenings are recommended starting at the age of 50.

It is therefore clear that there has been and remains today a long standing need for the identification of biomarkers which facilitate accurate and reliable diagnosis of colorectal cancer.

Multiple genetic and epigenetic mechanisms contribute to functional alterations of the tumor genome. Epigenetic modifications such as DNA methylation, have been found to occur already at the early stages of cancer development making them highly attractive for biomarker development. Hypermethylation within promoter regions is thought to induce tumor suppressor gene inactivation, whereas hypomethylation has been shown to lead to oncogene activation. In addition, hypomethylation of satellite regions might induce genomic instability.

The influence of copy number alterations (CNAs) on gene expression have mainly been shown to positively correlate, e.g., amplifications leading to an increase in gene expression. However, until now, the correlation between DNA methylation and gene expression, and in particular the influence of cancer differentially methylated regions (cDMRs) on gene expression patterns, have only been examined to a limited extent. Main limitations are the applied detection methods that allow the parallel analysis of methylation modifications only at selected genomic locations like e.g. CpG islands within promoter regions, or by the fact that studies have been performed on single genes. Moreover, long-range epigenetic mechanisms influence the cancer transcriptome. Such mechanisms, involving DNA methylation and histone modifications over large chromosomal stretches have been found in both copy-number dependent and independent regions.

To date, the most prominent differentially methylated genes in colorectal cancer and, therefore, be used as a biomarker for the detection of colorectal cancer, are, as recently reported, MLH1, APC, SEPT9 and ALX4 (Banerjee et al., Biomark Med 3, 397-410 (2009)). MLH1 and APC are not methylated at all or only in a distinct subgroup of cancers. SEPT9 and ALX4, which are located in a region that is subject to somatic copy number alterations (CNAs), show a variable performance for being used as a biomarker for colorectal cancer.

Accordingly, there is a need in the state of the art of studying genome-wide aberrant DNA methylation that can be associated with high confidence to colorectal cancer and identifying biomarkers for colorectal cancer diagnosis based on the epigenetic cancer information. The inventors hypothesized that enhanced biomarkers may be found in CNA-free regions, i.e. regions which are not subject to copy number alterations.

### SUMMARY OF THE INVENTION

The invention encompasses the identification and selection of novel genomic regions which are differentially methylated (differentially methylated regions, DMRs) in subjects with colorectal cancer compared to subjects without colorectal cancer so as to provide a simple and reliable test for diagnosing colorectal cancer. Nucleic acids which selectively hybridize to the genomic regions and products thereof are also encompassed within the scope of the invention as are compositions and kits containing said nucleic acids and nucleic acids for use in diagnosing colorectal cancer. Further encompassed by the invention is the use of nucleic acids each thereof selectively hybridizing to one of the genomic regions or products thereof to monitor disease progression or regression in a patient and the efficacy of therapeutic regimens.

For the first time the inventors have identified DMRs in a set of heterogeneous colorectal cancers by genome-wide approaches based on high throughput sequencing (methylated DNA immunoprecipitation, MeDIP-Seq) (Table 1) and thus, by quantifying the methylation status of specific genomic regions, permit the accurate and reliable diagnosis of colorectal cancer. The inventors found that CNAs influence DNA methylation patterns and mask the effects of DNA methylation marks on gene expression. They assume that CNAs do not only introduce a serious bias to biomarker discovery but also distort confidence of diagnosis. Therefore, in contrast to the known biomarkers, the herein described biomarkers are located in CNA-free regions.

The present invention, thus, contemplates a method for diagnosis of colorectal cancer, comprising the steps of analysing in a sample of a subject the DNA methylation status of at least one genomic region selected from the group of Table 1, wherein, if the at least one genomic region is differentially methylated, the sample is designated as colorectal cancer positive.

**Table 1: DMRs in colorectal cancer positive samples. Column 1: genomic region number according to GR No.; Column 2 to 4: locus in genome determined by the chromosome number and start and stop position of the sequence; Column 5: length of sequence; Column 6: associated or nearby gene; Column 7: differential methylation status found in colorectal cancer positive sample.**

| **GR NO** | **Chromosome** | **Start** | **Stop** | **Size of DMR** | **HUGO gene name** | **Differential methylation status +: hypermeth. -: hypometh.** |
|---|---|---|---|---|---|---|
| 1 | chr12 | 95941501 | 95943500 | 2000 | USP44 | + |
| 2 | chr2 | 115919751 | 115921250 | 1500 | DPP10 | + |
| 3 | chr3 | 192231751 | 192233750 | 2000 | FGF12; RP11-91M9.1 | + |
| 4 | chr1 | 99469501 | 99471250 | 1750 | RP11-254021.1; RP5-896L10.1 | + |
| 5 | chr10 | 7453501 | 7455500 | 2000 | | + |
| 6 | chr1 | 200010001 | 200011500 | 1500 | NR5A2 | + |
| 7 | chr12 | 3602001 | 3603000 | 1000 | PRMT8 | + |
| 8 | chr4 | 144621001 | 144622500 | 1500 | FREM3; RP13-578N3.3 | + |
| 9 | chr7 | 24322501 | 24325500 | 3000 | NPY | + |
| 10 | chr12 | 5018001 | 5020750 | 2750 | KCNA1 | + |
| 11 | chr3 | 192125501 | 192128750 | 3250 | FGF12 | + |
| 12 | chr6 | 73332001 | 73333500 | 1500 | KCNQ5; RP3-474G15.2 | + |
| 13 | chr1 | 111217001 | 111218500 | 1500 | KCNA3 | + |
| 14 | chr1 | 119527501 | 119528750 | 1250 | TBX15 | + |
| 15 | chr6 | 11143751 | 11144750 | 1000 | | - |
| 16 | chr10 | 115860001 | 115860500 | 500 | | - |
| 17 | chr5 | 1973501 | 1974500 | 1000 | | - |
| 18 | chr2 | 7100501 | 7101500 | 1000 | ACO 13460.1; AC017076.1; RNF144A | + |
| 19 | chr12 | 16757501 | 16758500 | 1000 | LMO3 | + |
| 20 | chr12 | 101916501 | 101917500 | 1000 | | - |
| 21 | chr2 | 68545751 | 68547500 | 1750 | CNRIP1 | + |
| 22 | chr6 | 36808251 | 36809250 | 1000 | | + |
| 23 | chr10 | 3805001 | 3806000 | 1000 | RP11-184A2.3 | - |
| 24 | chr2 | 22410751 | 22411500 | 750 | AC068044.1; AC068490.2 | - |
| 25 | chr7 | 6324251 | 6325000 | 750 | | - |
| 26 | chr2 | 69428251 | 69428750 | 500 | ANTXR1 | - |
| 27 | chr16 | 4000001 | 4001000 | 1000 | | - |
| 28 | chr1 | 38838251 | 38839000 | 750 | | - |
| 29 | chr4 | 188666001 | 188667000 | 1000 | | - |
| 30 | chr6 | 151561001 | 151561500 | 500 | AKAP12 | + |
| 31 | chr1 | 181638251 | 181639000 | 750 | CACNA1E | - |
| 32 | chr4 | 185000501 | 185001250 | 750 | | - |
| 33 | chr2 | 4816001 | 4816500 | 500 | | - |
| 34 | chr5 | 61041001 | 61041500 | 500 | CTD-2170G1.1 | - |
| 35 | chr3 | 196363251 | 196363750 | 500 | | - |
| 36 | chr4 | 183369001 | 183369750 | 750 | ODZ3 | + |
| 37 | chr1 | 158151001 | 158151750 | 750 | CD1D | + |
| 38 | chr7 | 145833251 | 145834000 | 750 | CNTNAP2 | - |
| 39 | chr1 | 170629751 | 170631250 | 1500 | | + |
| 40 | chr2 | 467501 | 469000 | 1500 | | + |
| 41 | chr16 | 72911501 | 72912000 | 500 | ATBF1 | - |
| 42 | chr22 | 48575751 | 48576250 | 500 | | - |
| 43 | chr3 | 113968001 | 113968500 | 500 | | - |
| 44 | chr2 | 55062251 | 55062750 | 500 | EML6 | - |
| 45 | chr6 | 7468251 | 7469250 | 1000 | | - |
| 46 | chr16 | 8172251 | 8172750 | 500 | | - |
| 47 | chr7 | 154657251 | 154657750 | 500 | DPP6 | - |
| 48 | chr1 | 244964001 | 244965000 | 1000 | | - |
| 49 | chr1 | 121260501 | 121261000 | 500 | | + |
| 50 | chr10 | 120683751 | 120684250 | 500 | | - |
| 51 | chr10 | 106905251 | 106905750 | 500 | SORCS3 | - |
| 52 | chr10 | 83633751 | 83635000 | 1250 | NRG3 | + |
| 53 | chr12 | 99288001 | 99289750 | 1750 | ANKS1B | + |
| 54 | chr12 | 103889251 | 103889750 | 500 | C12orf42 | + |
| 55 | chr16 | 22825251 | 22826750 | 1500 | HS3ST2 | + |
| 56 | chr19 | 58125501 | 58126500 | 1000 | ZNF134 | + |
| 57 | chr2 | 12858251 | 12859250 | 1000 | TRIB2 | + |
| 58 | chr22 | 25678501 | 25679750 | 1250 | CTA-221G9.9; RP3-462D8.2 | + |
| 59 | chr3 | 147124751 | 147125500 | 750 | ZIC1 | + |
| 60 | chr4 | 20254501 | 20256500 | 2000 | SLIT2 | + |
| 61 | chr5 | 72593751 | 72594750 | 1000 | | + |
| 62 | chr5 | 16179001 | 16181000 | 2000 | MARCH11; RP11-1902.2 | + |
| 63 | chr7 | 49814751 | 49815250 | 500 | VWC2 | + |
| 64 | chr8 | 54788751 | 54790500 | 1750 | RGS20 | + |

The invention also relates to a nucleic acid molecule that hybridizes under stringent conditions in the vicinity of one of the genomic regions according to numbers 1 to 64 of Table 1, wherein said vicinity is any position having a distance of up to 500 nt from the 3' or 5' end of said genomic region, wherein said vicinity includes the genomic region itself.

The invention further relates to the use of nucleic acids for the diagnosis of colorectal cancer.

Another subject of the present invention is a composition and a kit comprising one or more of said nucleic acids for the diagnosis of colorectal cancer.

The following detailed description of the invention refers, in part, to the accompanying drawings and does not limit the invention.

### DEFINITIONS

The following definitions are provided for specific terms which are used in the following.

The articles "a" and "an" are used herein to refer to one or to more than one (i.e. to at least one) of the grammatical object of the article. By way of example, "an element" means one element or more than one element. In contrast, "one" is used to refer to a single element.

As used herein, the term "amplified", when applied to a nucleic acid sequence, refers to a process whereby one or more copies of a particular nucleic acid sequence is generated from a nucleic acid template sequence, preferably by the method of polymerase chain reaction. Other methods of amplification include, but are not limited to, ligase chain reaction (LCR), polynucleotide-specific based amplification (NSBA), or any other method known in the art.

As used herein, the term "biomarker" refers to (a) a genomic region that is differentially methylated, e.g. hypermethylated or hypomethylated, or (b) a gene that is differentially expressed, wherein the status (hypo-/hypermethylation and/or up-/downregulated expression) of said biomarker can be used for diagnosing colorectal cancer or a stage of colorectal cancer as compared with those not having colorectal cancer. Within the context of the invention, a genomic region or parts thereof or fragment thereof are used as a biomarker for colorectal cancer. Within this context "parts of a genomic region" or a "fragment of a biomarker" means a portion of the genomic region or a portion of a biomarker comprising 1 or more CpG positions.

As used herein, the term "composition" refers to any mixture. It can be a solution, a suspension, liquid, powder, a paste, aqueous, non-aqueous or any combination thereof.

The term "CpG position" as used herein refers to a region of DNA where a cytosine nucleotide is located next to a guanine nucleotide in the linear sequence of bases along its length. "CpG" is shorthand for "C-phosphate-G", that is, cytosine and guanine separated by a phosphate, which links the two nucleosides together in DNA. Cytosines in CpG dinucleotides can be methylated to form 5-methylcytosine. This methylation of cytosines of CpG positions is a major epigenetic modification in multicellular organisms and is found in many human diseases including colorectal cancer.

As used herein, the term "diagnosis" refers to the identification of the disease (colorectal cancer) at any stage of its development, and also includes the determination of predisposition of a subject to develop the disease. In a preferred embodiment of the invention, diagnosis of colorectal cancer occurs prior to the manifestation of symptoms. Subjects with a higher risk of developing the disease are of particular concern. The diagnostic method of the invention also allows confirmation of colorectal cancer in a subject suspected of having colorectal cancer.

As used herein, the term "differential expression" refers to a difference in the level of expression of the RNA and/or protein products of one or more biomarkers, as measured by the amount or level of RNA or protein. In reference to RNA, it can include difference in the level of expression of mRNA, and/or one or more spliced variants of mRNA and/or the level of expression of small RNA (miRNA) of the biomarker in one sample as compared with the level of expression of the same one or more biomarkers of the invention as measured by the amount or level of RNA, including mRNA, spliced variants of mRNA or miRNA in a second sample or with regard to a threshold value. "Differentially expressed" or "differential expression" can also include a measurement of the protein, or one or more protein variants encoded by the inventive biomarker in a sample as compared with the amount or level of protein expression, including one or more protein variants of the biomarker in another sample or with regard to an threshold value. Differential expression can be determined, e.g. by array hybridization, next generation sequencing, RT-PCR or an immunoassay and as would be understood by a person skilled in the art.

As used herein, the term "differential methylation" or "aberrant methylation" refers to a difference in the level of DNA/cytosine methylation in a colorectal cancer positive sample as compared with the level of DNA methylation in a colorectal cancer negative sample. The "DNA methylation status" is interchangeable with the term "DNA methylation level" and can be assessed by determining the ratio of methylated and non-methylated DNA of a genomic region or a portion thereof and is quoted in percentage. For example, the methylation status of a sample is 60% if 60 % of the analysed genomic region of said sample is methylated and 40 % of the analysed genomic region of said sample is not methylated.

The methylation status can be classified as increased ("hypermethylated"), decreased ("hypomethylated") or normal as compared to a benign sample. The term "hypermethylated" is used herein to refer to a methylation status of at least more than 10% methylation in the tumour in comparison to the maximal possible methylation value in the normal, most preferably above 15 %, 20 %, 25% or 30% of the maximum values. For comparison, a hypomethylated sample has a methylation status of less than 10%, most preferably below 15%, 20%, 25% or 30% of the minimal methylation value in the normal.

The percentage values can be estimated from bisulphite mass spectrometry data (Epityper). Being obvious to the skilled person, the measurement error of the method (ca 5 %) and the error coming from preparation of the sample must be considered. Particularly, the aforementioned values assume a sample which is not contaminated with other DNA (e.g. micro dissected sample) than those coming from colorectal cells. As would be understood to the skilled person the values must be recalculated for contaminated samples (e.g. macro dissected samples). If desired, other methods can be used, such as the methods described in the following for analyzing the methylation status. However, the skilled person readily knows that the absolute values as well as the measurement error can differ for different methods and he knows how to compensate for this.

The term, "analyzing the methylation status" or "measuring the methylation", as used herein, relates to the means and methods useful for assessing and quantifying the methylation status. Useful methods are bisulphite-based methods, such as bisulphite-based mass spectrometry, bisulphite-based sequencing methods or enrichment methods such as MeDIP-Sequencing methods. Likewise, DNA methylation can also be analyzed directly via single-molecule real-time sequencing, single-molecule bypass kinetics and single-molecule nanopore sequencing.

As used herein, the term "genomic region" refers to a sector of the genomic DNA of any chromosome that can be subject to differential methylation within said sector and may be used as a biomarker for the diagnosis of colorectal cancer according to the invention. For example, each sequence listed in Table 1 and Table 2 with the corresponding genomic region numbers 1 to 64 is a genomic region according to the invention. A genomic region can comprise the full sequence or parts thereof provided that at least one CpG position is comprised by said part. Preferably, said part comprises between 1 to 15 CpG positions. In another embodiment, the genomic region can comprise at least 1, 2, 3, 4, 5, 6, 7, 8, 9, 10, 11, 12, 13, 14, or 15 CpG positions.

Genomic regions that occur in the vicinity of genes may be associated with the names of those genes for descriptive purpose. This may not mean, that the genomic region comprises all or a part of that gene or functional elements of it. In case of doubt, solely the locus and/or the sequence shall be used.

As used herein, the term "in the vicinity of a genomic region" refers to a position outside or within said genomic region. As would be understood to a person skilled in the art the position may have a distance up to 500 nucleotides (nt), 400 nt, 300 nt, 200 nt, 100 nt, 50 nt, 20 nt or 10 nt from the 5' or 3' end of the genomic region. Alternatively, the position is located at the 5' or 3' end of said genomic region, or, the position is within said genomic region.

The term "genomic region specific primers" as used herein refers to a primer pair hybridizing to a flanking sequence of a target sequence to be amplified. Such a sequence starts and ends in the vicinity of a genomic region. In one embodiment, the target sequence to be amplified comprises the whole genomic region and its complementary strand. In a preferred embodiment, the target sequence comprises 1, 2, 3, 4, 5, 6, 7, 8, 9, 10, 11, 12, 13, 14, 15 or even more CpG positions of the genomic region and the complementary strand thereof. In general, the hybridization position of each primer of the primer pair can be at any position in the vicinity of a genomic region provided that the target sequence to be amplified comprises at least one CpG position of said genomic region. As would be obvious to the skilled person, the sequence of the primer depends on the hybridization position and on the method for analyzing the methylation status, e.g. if a bisulphite based method is applied, part of the sequence of the hybridization position may be converted by said bisulphite. Therefore, in one embodiment, the primers may be adapted accordingly to still enable or disable hybridization (e.g. in methylation specific PCR).

The term "genomic region specific probe" as used herein refers to a probe that selectively hybridizes to a genomic region. In one embodiment a genomic region specific probe can be a probe labelled, for example with a fluorophore and a quencher, such as a TaqMan® probe or a Molecular Beacons probes. In a preferred embodiment, the probe can hybridize to a position of the genomic region that can be subject to hypermethylation according to the inventive method. Hereby, the probe hybridizes to positions with either a methylated CpG or a unmethylated CpG in order to detect methylated or unmethylated CpGs. In a preferred embodiment, two probes are used, e.g. in a methylight (qPCR assay) assay. The first probe hybridizes only to positions with a methylated CpG, the second probe hybridizes only to positions with a unmethylated CpG, wherein the probes are differently labelled and, thus, allow for discrimination between unmethylated and methylated sites in the same sample.

As used herein, the terms "hybridizing to" and ``hybridization" are interchangeable used with the term "specific for" and refer to the sequence specific non-covalent binding interactions with a complementary nucleic acid, for example, interactions between a target nucleic acid sequence and a target specific nucleic acid primer or probe. In a preferred embodiment a nucleic acid, which hybridizes is one which hybridizes with a selectivity of greater than 70%, greater than 80%, greater than 90% and most preferably of 100% (i.e. cross hybridization with other DNA species preferably occurs at less than 30%, less than 20%, less than 10%). As would be understood to a person skilled in the art, a nucleic acid, which "hybridizes" to the DNA product of a genomic region of the invention can be determined taking into account the length and composition.

As used herein, "isolated" when used in reference to a nucleic acid means that a naturally occurring sequence has been removed from its normal cellular (e.g. chromosomal) environment or is synthesised in a non-natural environment (e.g. artificially synthesised). Thus, an "isolated" sequence may be in a cell-free solution or placed in a different cellular environment.

As used herein, a "kit" is a packaged combination optionally including instructions for use of the combination and/or other reactions and components for such use.

As used herein, "nucleic acid(s)" or "nucleic acid molecule" generally refers to any ribonucleic acid or deoxyribonucleic acid, which may be unmodified or modified DNA. "Nucleic acids" include, without limitation, single- and double-stranded nucleic acids. As used herein, the term "nucleic acid(s)" also includes DNA as described above that contain one or more modified bases. Thus, DNA with backbones modified for stability or for other reasons are "nucleic acids". The term "nucleic acids" as it is used herein embraces such chemically, enzymatically or metabolically modified forms of nucleic acids, as well as the chemical forms of DNA characteristic of viruses and cells, including for example, simple and complex cells.

The term "primer", as used herein, refers to an nucleic acid, whether occurring naturally as in a purified restriction digest or produced synthetically, which is capable of acting as a point of initiation of synthesis when placed under conditions in which synthesis of a primer extension product, which is complementary to a nucleic acid strand, is induced, i.e., in the presence of nucleotides and an inducing agent such as a DNA polymerase and at a suitable temperature and pH. The primer may be either single-stranded or double-stranded and must be sufficiently long to prime the synthesis of the desired extension product in the presence of the inducing agent. The exact length of the primer will depend upon many factors, including temperature, source of primer and the method used. For example, for diagnostic applications, depending on the complexity of the target sequence, the nucleic acid primer typically contains 15-25 or more nucleotides, although it may contain fewer nucleotides. The factors involved in determining the appropriate length of primer are readily known to one of ordinary skill in the art. In general, the design and selection of primers embodied by the instant invention is according to methods that are standard and well known in the art, see Dieffenbach, C. W., Lowe, T. M. J., Dveksler, G. S. (1995) General Concepts for PCR Primer Design. In: PCR Primer, A Laboratory Manual (Eds. Dieffenbach, C. W, and Dveksler, G. S.) Cold Spring Harbor Laboratory Press, New York, 133-155; Innis, M. A., and Gelfand, D. H. (1990) Optimization of PCRs. In: PCR protocols, A Guide to Methods and Applications (Eds. Innis, M. A., Gelfand, D. H., Sninsky, J. J;, and White, T. J.) Academic Press, San Diego, 3-12; Sharrocks, A. D. (1994) The design of primers for PCR. In: PCR Technology, Current Innovations (Eds. Griffin, H. G., and Griffin, A. M, Ed.) CRC Press, London, 5-11.

As used herein, the term "probe" means nucleic acid and analogs thereof and refers to a range of chemical species that recognise polynucleotide target sequences through hydrogen bonding interactions with the nucleotide bases of the target sequences. The probe or the target sequences may be single- or double-stranded DNA. A probe is at least 8 nucleotides in length and less than the length of a complete polynucleotide target sequence. A probe may be 10, 20, 30, 50, 75, 100, 150, 200, 250, 400, 500 and up to 2000 nucleotides in length. Probes can include nucleic acids modified so as to have a tag which is detectable by fluorescence, chemiluminescence and the like ("labelled probe"). The labelled probe can also be modified so as to have both a detectable tag and a quencher molecule, for example Taqman® and Molecular Beacon® probes. The nucleic acid and analogs thereof may be DNA, or analogs of DNA, commonly referred to as antisense oligomers or antisense nucleic acid. Such DNA analogs comprise but are not limited to 2-'O-alkyl sugar modifications, methylphosphonate, phosphorothiate, phosphorodithioate, formacetal, 3'-thioformacetal, sulfone, sulfamate, and nitroxide backbone modifications, and analogs wherein the base moieties have been modified. In addition, analogs of oligomers may be polymers in which the sugar moiety has been modified or replaced by another suitable moiety, resulting in polymers which include, but are not limited to, morpholino analogs and peptide nucleic acid (PNA) analogs (Egholm, et al. Peptide Nucleic Acids (PNA)-Oligonucleotide Analogues with an Achiral Peptide Backbone, (1992)).

The term "sample" or "biological sample" is used herein to refer to colorectal tissue, blood, urine, semen, colorectal secretions or isolated colorectal cells originating from a subject, preferably from colorectal tissue, colorectal secretions or isolated colorectal cells, most preferably to colorectal tissue.

As used herein, the term "DNA sequencing" or "sequencing" refers to the process of determining the nucleotide order of a given DNA fragment. As known to those skilled in the art, sequencing techniques comprise sanger sequencing and next-generation sequencing, such as 454 pyrosequencing, Illumina (Solexa) sequencing and SOLiD sequencing.

The term "bisulphite sequencing" refers to a method well-known to the person skilled in the art comprising the steps of (a) treating the DNA of interest with bisulphite, thereby converting non-methylated cytosines to uracils and leaving methylated cytosines unaffected and (b) sequencing the treated DNA, wherein the existence of a methylated cytosine is revealed by the detection of a non-converted cytosine and the absence of a methylated cytosine is revealed by the detection of a thymine.

As used herein, the terms "subject" and "patient" are used interchangeably to refer to an animal (e.g., a mammal, a fish, an amphibian, a reptile, a bird and an insect). In a specific embodiment, a subject is a mammal (e.g., a non-human mammal and a human). In another embodiment, a subject is a primate (e.g., a chimpanzee and a human). In another embodiment, a subject is a human. In another embodiment, the subject is a male human with or without colorectal cancer.

### DETAILED DESCRIPTION OF THE INVENTION

The practice of the present invention employs in part conventional techniques of molecular biology, microbiology and recombinant DNA techniques, which are within the skill of the art. Such techniques are explained fully in the literature. See, e.g., Sambrook, Fritsch & Maniatis, 1989, Molecular Cloning: A Laboratory Manual, Second Edition; Oligonucleotide Synthesis (M. J. Gait, ed., 1984); Nucleic Acid Hybridization (B. D. Harnes & S. J. Higgins, eds., 1984); A Practical Guide to Molecular Cloning (B. Perbal, 1984); and a series, Methods in Enzymology (Academic Press, Inc.); Short Protocols In Molecular Biology, (Ausubel et al., ed., 1995). All patents, patent applications, and publications mentioned herein, both supra and infra, are hereby incorporated by reference in their entireties.

The invention as disclosed herein identifies genomic regions that are useful in diagnosing colorectal cancer. By definition, the identified genomic regions are biomarkers for colorectal cancer. In order to use these genomic regions (as biomarkers), the invention teaches the analysis of the DNA methylation status of said genomic regions. The invention further encompasses genomic region specific nucleic acids. The invention further contemplates the use of said genomic region specific nucleic acids to analyse the methylation status of a genomic region, either directly or indirectly by methods known to the skilled person and explained herein. The invention further discloses a composition and kit comprising said nucleic acids for the diagnosis of colorectal cancer.

To address the need in the art for a more reliable diagnosis of colorectal cancer, the peculiarities of the DNA methylation status across the whole genome of colorectal cancer positive samples were examined in comparison to colorectal cancer negative samples. The inventors found genomic regions, that are subject to an differential methylation status. Therefore, the invention teaches the analysis of those genomic regions that are differentially methylated in samples from patients having colorectal cancer. Superior to current diagnostic methods, the invention discloses genomic regions, wherein most astonishingly a single genomic region is able to diagnose colorectal cancer with high confidence. If at least one genomic region is differentially methylated, the sample can be designated as colorectal cancer positive. The inventors found that the identified genomic regions are located in CNA-free regions. CNAs are alterations of the DNA of a genome that results in the cell having an abnormal number of copies of one or more sections of the DNA. The inventors partly attribute the superiority of the new biomarkers to the fact that all biomarkers are located in CNA-free regions and, therefore, are not subject to distorting effects of CNA regions.

Accordingly, the invention relates to a method for diagnosis of colorectal cancer, comprising the steps of analysing in a sample of a subject the DNA methylation status of at least one genomic region selected from the group of Table 1, wherein, if the at least one genomic region is differentially methylated, the sample is designated as colorectal cancer positive. In a preferred embodiment, the genomic region to be analysed is selected from the group of genomic region numbers 1 to 30. In a more preferred embodiment, the genomic region to be analysed is selected from the group of genomic region numbers 1 to 20. In an even more preferred embodiment, the genomic region to be analysed is selected from the group of genomic region numbers 1 to 10. In an even more preferred embodiment, the genomic region to be analysed is selected from the group of GR NOs. 1 to GR NOs 7. In an even more preferred embodiment, the genomic region to be analysed is selected from the group of GR NO. 1 to GR NO. 5. In the most preferred embodiment, the genomic region to be analysed is selected from the group of genomic region number 1.

In certain embodiments of the invention disclosed herein the at least one genomic region is selected from a subgroup of Table 1, wherein the at least one genomic region is hypermethylated or hypomethylated depending on the subgroup selected. A first subgroup contains genomic regions that are hypermethylated in colorectal cancer, i.e. numbers 1 - 14, 18, 19, 21, 22, 30, 36, 37, 39, 40, 49 and 52 - 64. A second subgroup contains genomic regions that are hypomethylated in colorectal cancer, i.e.numbers 15 - 17, 20, 23 - 29, 31 - 35, 38, 41 -48, 50 and 51.

Significantly, the inventors found that a minimum of one genomic region is sufficient to accurately discriminate between malignant and benign tissues. The extension with additional sites even increases the discriminatory potential of the marker set. Thus, in another embodiment, the invention relates to a method, wherein the methylation status of a further genomic region and/or a further biomarker is analysed.

In one embodiment of the invention, one or more known colorectal cancer biomarker are additionally analysed. Such colorectal cancer biomarkers can be a gene, e.g. encoding for SEPT9, ALX4, BRAF, MLH1, TMEFF2, BMP3, EYA2,or APC. Such biomarkers can also be based on gene expression, e.g. of said encoding genes. The analysis of the biomarkers within this context can be the analysis of the methylation status, the analysis of the gene expression (mRNA), or the analysis of the amount or concentration or activity of protein.

In another embodiment one or more further genomic region according to the invention is analysed. For example, a total of 2, 3, 4, 5, 6, 7, 8, 9 or 10 genomic regions selected from the group of Table 1 is analysed. In a specific embodiment, at least two genomic regions are analysed: The first genomic region has the sequence according to GR NO. 1 and the second genomic region is selected from the group of Table 1, or the first genomic region has the sequence according to GR NO. 2 and the second genomic region is selected from the group of Table 1, or the first genomic region has the sequence according to GR NO. 3 and the second genomic region is selected from the group of Table 1, or the first genomic region has the sequence according to GR NO. 4 and the second genomic region is selected from the group of Table 1, or the first genomic region has the sequence according to GR NO. 5 and the second genomic region is selected from the group of Table 1. However, it is to be understood that the invention is neither restricted to a specific genomic region nor to a specific combination. Accordingly, any genomic region or combination of genomic regions according to Table 1 may be used herein. As will be understood by the skilled person the presence of differential methylation of each of said biomarkers in the biological sample is determined; and the presence of differential methylation of said biomarkers is correlated with a positive indication of colorectal cancer in said subject.

The method is particularly useful for early diagnosis of colorectal cancer. The method is useful for further diagnosing patients having symptoms associated with colorectal cancer. The method of the present invention can further be of particular use with patients having an enhanced risk of developing colorectal cancer (e.g., patients having a familial history of colorectal cancer and patients identified as having a mutant oncogene). The method of the present invention may further be of particular use in monitoring the efficacy of treatment of a colorectal cancer patient (e.g. the efficacy of chemotherapy).

In one embodiment of the method, the sample comprises cells obtained from a patient. The cells may be found in a colorectal tissue sample collected, for example, by a colorectal tissue biopsy or histology section, or a bone marrow biopsy if metastatic spreading has occurred. In another embodiment, the patient sample is a colorectal-associated body fluid. Such fluids include, for example, blood fluids, lymph, and feces. From the samples cellular or cell free DNA is isolated using standard molecular biological technologies and then forwarded to the analysis method.

In order to analyse the methylation status of a genomic region, conventional technologies can be used.

Either the DNA of interest may be enriched, for example by methylated DNA immunoprecipitation (MeDIP) followed by real time PCR analyses, array technology, or next generation sequencing. Alternatively, the methylation status of the DNA can be analysed directly or after bisulphite treatment.

In one embodiment, bisulphite-based approaches are used to preserve the methylation information. Therefore, the DNA is treated with bisulphite, thereby converting non-methylated cytosine residues into uracil while methylated cytosines are left unaffected. This selective conversion makes the methylation easily detectable and classical methods reveal the existence or absence of DNA (cytosine) methylation of the DNA of interest. The DNA of interest may be amplified before the detection if necessary. Such detection can be done by mass spectrometry or, the DNA of interest is sequenced. Suitable sequencing methods are direct sequencing and pyrosequencing. In another embodiment of the invention the DNA of interest is detected by a genomic region specific probe that is selective for that sequence in which a cytosine was either converted or not converted. Other techniques that can be applied after bisulphite treatment are for example methylation-sensitive single-strand conformation analysis (MS-SSCA), high resolution melting analysis (HRM), methylation-sensitive single-nucleotide primer extension (MS-SnuPE), methylation specific PCR (MSP) and base-specific cleavage.

In an alternative embodiment the methylation status of the DNA is analysed without bisulphite treatment, such as by methylation specific enzymes or by the use of a genomic region specific probe or by an antibody, that is selective for that sequence in which a cytosine is either methylated or non-methylated.

In a further alternative, the DNA methylation status can be analysed via single-molecule real-time sequencing, single-molecule bypass kinetics and single-molecule nanopore sequencing. These techniques, which are within the skill of the art, are fully explained in: Flusberg et al. Direct detection of DNA methylation during single-molecule, real-time sequencing. Nature methods 7(6): 461-467. 2010; Summerer. High-Througput DNA Sequencing Beyond the Four-Letter Code: Epigenetic Modifications Revealed by Single-Molecule Bypass Kinetics. ChemBioChem 11: 2499-2501. 2010; Clarke et al. Continuous base identification for single-molecule nanopore DNA sequencing. Nature Nanotechnology 4: 265-270. 2009; Wallace et al. Identification of epigenetic DNA modifications with a protein nanopore. Chemical Communication 46:8195-8197, which are hereby incorporated by reference in their entireties.

To translate the raw data generated by the detection assay (e.g. a nucleotide sequence) into data of predictive value for a clinician, a computer-based analysis program can be used.

The profile data may be prepared in a format suitable for interpretation by a treating clinician. For example, rather than providing raw nucleotide sequence data or methylation status, the prepared fonnat may represent a diagnosis or risk assessment (e.g. likelihood of cancer being present or the subtype of cancer) for the subject, along with recommendations for particular treatment options.

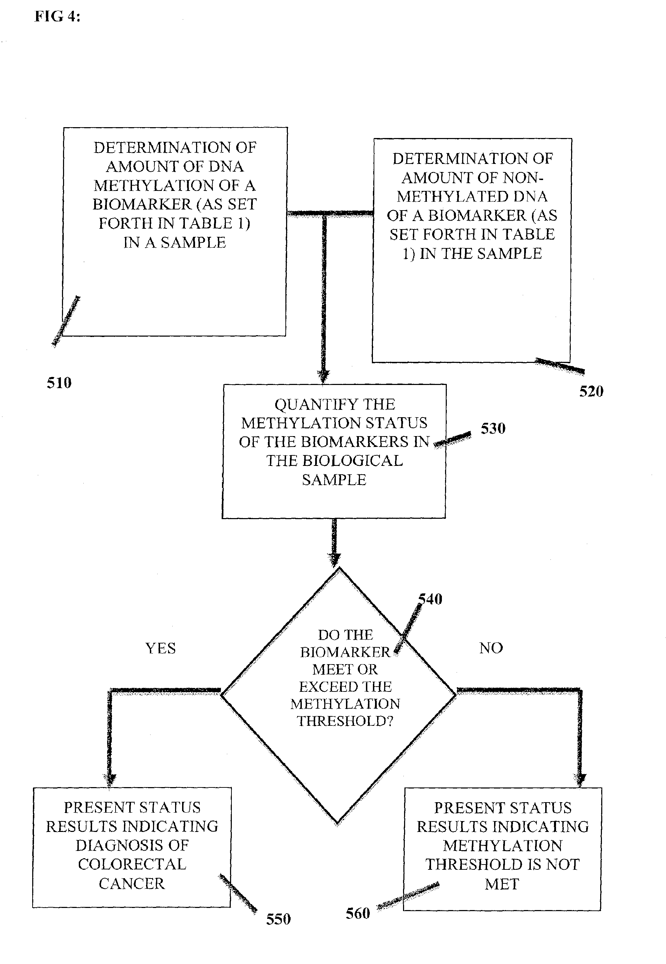

In one embodiment of the present invention, a computing device comprising a client or server component may be utilized. Figure 4 is an exemplary diagram of a client/server component, which may include a bus **210**, a processor **220**, a main memory **230**, a read only memory (ROM) **240**, a storage device **250**, an input device **260**, an output device **270**, and a communication interface **280**. Bus 210 may include a path that permits communication among the elements of the client/server component.

Processor **220** may include a conventional processor or microprocessor, or another type of processing logic that interprets and executes instructions. Main memory **230** may include a random access memory (RAM) or another type of dynamic storage device that stores information and instructions for execution by processor 220. ROM **240** may include a conventional ROM device or another type of static storage device that stores static information and instructions for use by processor **220**. Storage device **250** may include a magnetic and/or optical recording medium and its corresponding drive.

Input device 260 may include a conventional mechanism that permits an operator to input information to the client/server component, such as a keyboard, a mouse, a pen, voice recognition and/or biometric mechanisms, etc. Output device **270** may include a conventional mechanism that outputs information to the operator, including a display, a printer, a speaker, etc. Communication interface **280** may include any transceiver-like mechanism that enables the client/server component to communicate with other devices and/or systems. For example, communication interface **280** may include mechanisms for communicating with another device or system via a network.

As will be described in detail below, the client/server component, consistent with the principles of the invention, may perform certain measurement determinations of methylation, calculations of methylation status, and/or correlation operations relating to the diagnosis of colorectal cancer. It may further optionally output the presentation of status results as a result of the processing operations conducted. The client/server component may perform these operations in response to processor **220** executing software instructions contained in a computer-readable medium, such as memory **230**. A computer-readable medium may be defined as a physical or logical memory device and/or carrier wave.

The software instructions may be read into memory **230** from another computer-readable medium, such as data storage device **250**, or from another device via communication interface **280**. The software instructions contained in memory **230** may cause processor **220** to perform processes that will be described later. Alternatively, hardwired circuitry may be used in place of or in combination with software instructions to implement processes consistent with the principles of the invention. Thus, implementations consistent with the principles of the invention are not limited to any specific combination of hardware circuitry and software.

FIG. 5 is a flowchart of exemplary processing of methylation status for biomarkers present in biological samples according to an implementation consistent with the principles of the present invention. Processing may begin with quantifying the methylation **510** and non-methylation **520** of the DNA of a biological sample for a biomarker of Table 1 or, in an alternative embodiment, for more than a single biomarker if desired (see above). The processor may then quantify the methylation status **530,** as described above, as the ratio of methylated DNA to non-methylated of the biological sample for the biomarker(s). The methylation status may then be evaluated either via a computing device **540** or by human analysis to determine if the biomarker(s) meet or exceed a predetermined methylation threshold. If the threshold is met or exceeded, the computing device may then, optionally, present a status result indicating a positive diagnosis of colorectal cancer **550.** Alternatively, if the threshold is not met, then the computing device may, optionally, present a status result indicating that the threshold is not satisfied **560.** It is noted that the output displaying results may differ depending on the desired presentation of results. For example, the output may be quantitative in nature, *e.g.*, displaying the measurement values of each of the biomarkers in relation to the predetermined methylation threshold value. The output may be qualitative, *e.g.*, the display of a color or notation indicating a positive result for colorectal cancer, or a negative results for colorectal cancer, as the case may be. Notably, this process may be repeated multiple times using different genomic regions, as set forth in Table 1. The computing device may alternatively be programmed to permit the analysis of more than one genomic region at one time.

In some embodiments, the results are used in a clinical setting to determine a further diagnostic (e.g., additional further screening (e.g., other markers or diagnostic biopsy) course of action. In other embodiments, the results are used to determine a treatment course of action (e.g., choice of therapies or watchful waiting).

The inventors surprisingly found that the methylation status within a genomic region according to the invention is almost constant, leading to a uniform distribution of either hyper- or hypomethylated CpG positions within said genomic region. In one embodiment of the invention, all CpG positions of a genomic region are analysed. In a specific embodiment, CpG positions in the vicinity of the genomic region may be analysed. In an alternative embodiment, a subset of CpG positions of a genomic region is analysed. Ideally, 1, 2, 3, 4, 5, 6, 7, 8, 9, 10 GpG positions of a genomic region are analysed. Therefore, a preferred embodiment of the invention relates to a method, wherein analysing the methylation status of a genomic region means analysing the methylation status of at least one CpG position per genomic region.

In a preferred embodiment the invention relates to a method, wherein the methylation status is analysed by non-methylation-specific PCR based methods followed by sequencing, methylation-based methods such as methylation sensitive PCR, EpiTyper and Methylight assays or enrichment-based methods such as MeDIP-Seq. In an alternative embodiment of the present invention, the DNA methylation is assessed by methylation-specific restriction analysis.

In a preferred embodiment of the invention Epityper® and Methylight® assays may be used for the analysis of the methylation status.

The invention also relates to a nucleic acid molecule that hybridizes under stringent conditions in the vicinity of one of the genomic regions according to SEQ ID NO. 1 to SEQ ID NO. 64, wherein said vicinity relates to a position as defined above. In one embodiment said nucleic acid is 15 to 100 nt in length. In a preferred embodiment said nucleic acid is 15 to 50 nt, in a more preferred embodiment 15 to 40 nt in length.

In another embodiment said nucleic acid is a primer. The inventive primers being specific for a genomic region can be used for the analysis methods of the DNA methylation status. Accordingly, they are used for amplification of a sequence comprising the genomic region or parts thereof in the inventive method for the diagnosis of PC. Within the context of the invention, the primers selectively hybridizes in the vicinity of the genomic region as defined above.

Primers may be prepared using any suitable method, such as, for example, the phosphotriester and phosphodiester methods or automated embodiments thereof. In one such automated embodiment diethylophosphoramidites are used as starting materials and may be synthesized as described by Beaucage et al., Tetrahedron Letters, 22:1859-1862 (1981), which is hereby incorporated by reference. One method for synthesizing oligonucleotides on a modified solid support is described in U.S. Pat. No. 4,458,006, which is hereby incorporated by reference. It is also possible to use a primer which has been isolated from a biological source (such as a restriction endonuclease digest).

The methylation status of a genomic region may be detected indirectly (e.g. by bisulphite sequencing) or directly by using a genomic region specific probe, e.g. in a methylight assay. Thus, the present invention also relates to said nucleic acid being a probe. In a preferred embodiment of the present invention the probe is labelled.

Said probes can also be used in techniques such as quantitative real-time PCR (qRT-PCR), using for example SYBR®Green, or using TaqMan® or Molecular Beacon techniques, where the nucleic acids are used in the form of genomic region specific probes, such as a TaqMan labelled probe or a Molecular Beacon labelled probe. Within the context of the invention, the probe selectively hybridizes to the genomic region as defined above. Additionally, in qRT-PCR methods a probe can also hybridize to a position in the vicinity of a genomic region.

Current methods for the analysis of the methylation status require a bisulphite treatment a priori, thereby converting non-methylated cytosines to uracils. To ensure the hybridization of the genomic region specific nucleic acid of the invention to the bisulphite treated DNA, the nucleotide sequence of the nucleic acid may be adapted. For example, if it is desired to design nucleic acids being specific for a sequence, wherein a cytosine is found to be differentially methylated, that genomic region specific nucleic acid may have two sequences: the first bearing an adenine, the second bearing an guanine at that position which is complementary to the cytosine nucleotide in the sequence of the genomic region. The two forms can be used in an assay to analyse the methylation status of a genomic region such that they are capable of discriminating between methylated and non-methylated cytosines. Depending on the analysis method and the sort of nucleic acid (primer/probe), only one form or both forms of the genomic region specific nucleic acid can be used within the assay. Thus, in an alternative embodiment of the present invention the nucleic acid hybridizes under stringent conditions in said vicinity of one of the genomic regions after a bisulphite treatment.

The present invention also relates to the use of genomic region specific nucleic acids for the diagnosis of colorectal cancer.

The present invention also comprises the use of an antibody that is specific for a genomic region for the diagnosis of colorectal cancer.

Such antibody may preferably bind to methylated nucleotides. In another embodiment the antibody preferably binds to non-methylated nucleotides. The antibody can be labelled and/or used in an assay that allows the detection of the bound antibody, e.g. ELISA.

The nucleic acid or antibody for performing the method according to the invention is advantageously formulated in a stable composition. Accordingly, the present invention relates to a composition for the diagnosis of colorectal cancer comprising said nucleic acid or antibody.

The composition may also include other substances, such as stabilizers.

The invention also encompasses a kit for the diagnosis of colorectal cancer comprising the inventive nucleic acid or antibody as described above.

The kit may comprise a container for a first set of genomic region specific primers. In a preferred embodiment, the kit may comprise a container for a second set of genomic region specific primers. In a further embodiment, the kit may also comprise a container for a third set of genomic region specific primers. In a further embodiment, the kit may also comprise a container for a fourth set of genomic region specific primers, and so forth.

The kit may also comprise a container for bisulphite, which may be used for a bisulphite treatment of the genomic region of interest.

The kit may also comprise genomic region specific probes.

The kit may comprise containers of substances for performing an amplification reaction, such as containers comprising dNTPs (each of the four deoxynucleotides dATP, dCTP, dGTP, and dTTP), buffers and DNA polymerase.

The kit may also comprise nucleic acid template(s) for a positive control and/or negative control reaction. In one embodiment, a polymerase is used to amplify a nucleic acid template in PCR reaction. Other methods of amplification include, but are not limited to, ligase chain reaction (LCR), or any other method known in the art.

The kit may also comprise containers of substances for performing a sequencing reaction, for example pyrosequencing, such as DNA polymerase, ATP sulfurylase, luciferase, apyrase, the four deoxynucleotide triphosphates (dNTPs) and the substrates adenosine 5' phosphosulfate (APS) and luciferin.

### FIGURE CAPTIONS

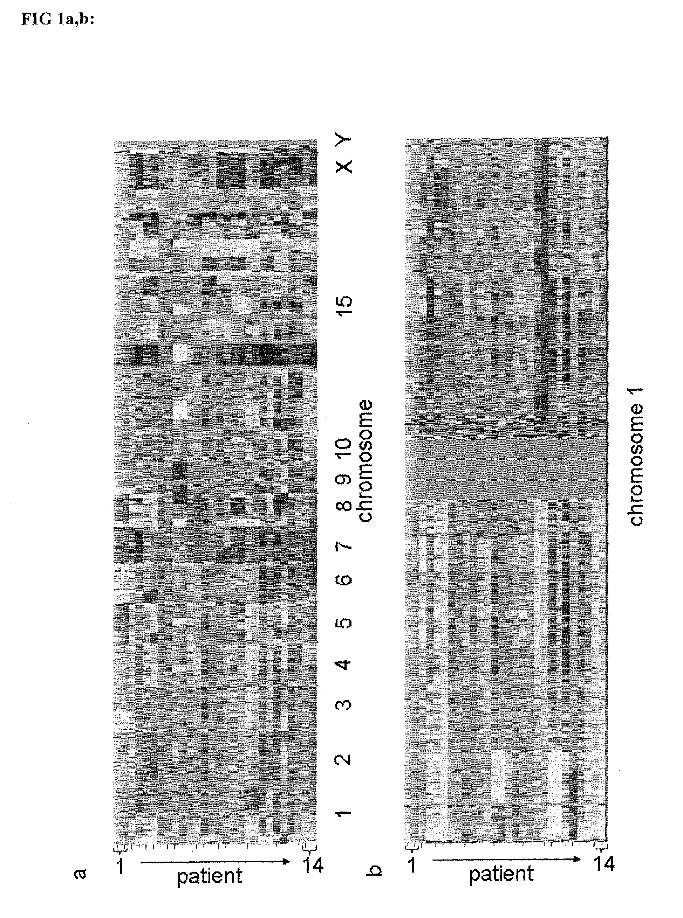

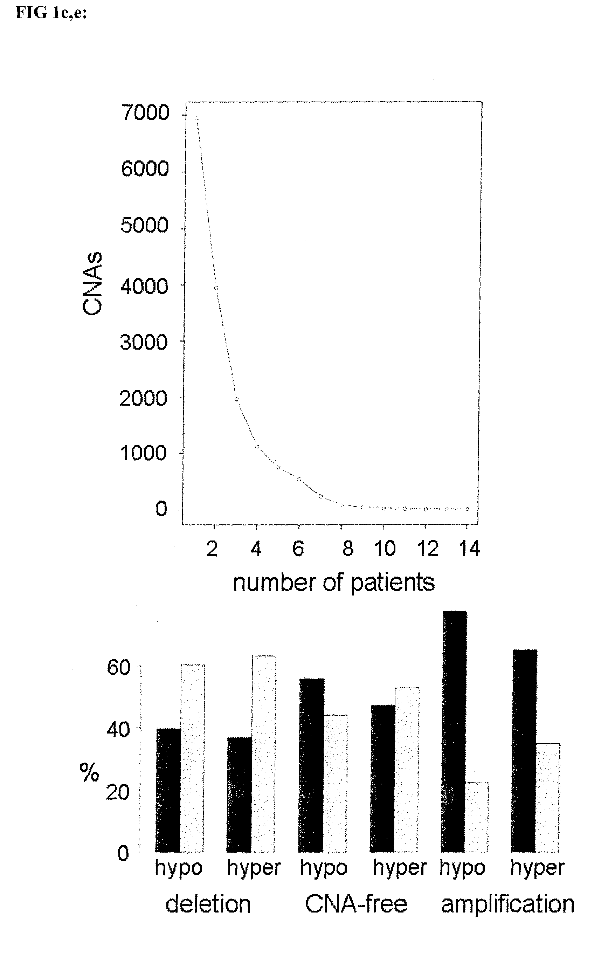

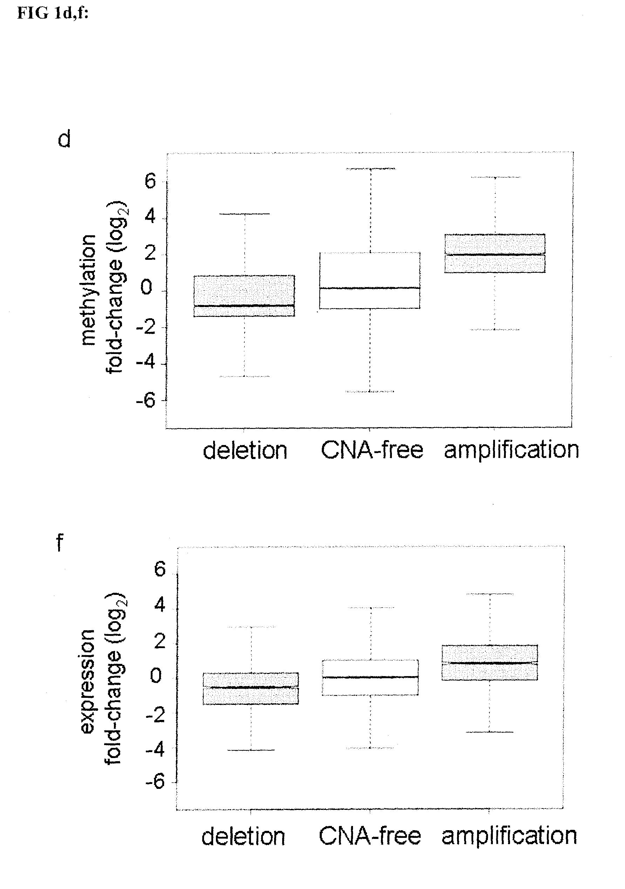

**Figure 1****: Impact of CNA status on methylation and gene expression.** (a) Global patterns of DNA methylation and CNAs. For each patient (P1-P14) a color-coded representation of methylation (orange labelled rows) and CNA fold-changes (green labelled rows) is shown for 5 million bp adjacent windows across all chromosomes (log2-scale). Yellow colors refer to deletions and hypomethylations and blue colors refer to amplifications and hypermethylations respectively when comparing tumor versus normal tissue. (b) Magnification of chromosome 1 with windows of 0.5 million bp length using the same color-coding. (c) Distribution of somatic CNAs (Y-axis) across all patients (X-axis). (d) Correlation of methylation fold-changes (Y-axis, log2-scale) and CNA status (X-axis). DMRs (tumor versus normal) from all patients were sampled and divided in three groups: DMRs that fall into deletions, amplifications and CNA-free regions. Box plots show the median methylation fold-changes for the three groups and the interquartile range. (e) Correlation of gene expression, DNA methylation and CNAs. Differentially expressed genes were divided into three groups (deletions, CNA-free and amplifications). Bars show the proportion of hyper- and hypomethylated proximal promoter regions (-1 kb to + 0.5kb) within these groups. For each combination of copy number and promoter methylation status the number of up-regulated (dark grey) - and down-regulated (light grey) genes were calculated. For promoters localized in CNA free regions significant correlations between hypermethylation and decreased gene expression as well as between hypomethylation and increased gene expression was observed (Fisher's exact test p-value<0.006). (f) Correlation of expression fold-changes (Y-axis, log2-scale) and CNA status (X-axis). Gene expression values (tumor versus normal) for P12 were divided in three groups: genes that fall into deletions, amplifications and CNA-free regions. Box plots show the median values for the three groups and the interquartile range.

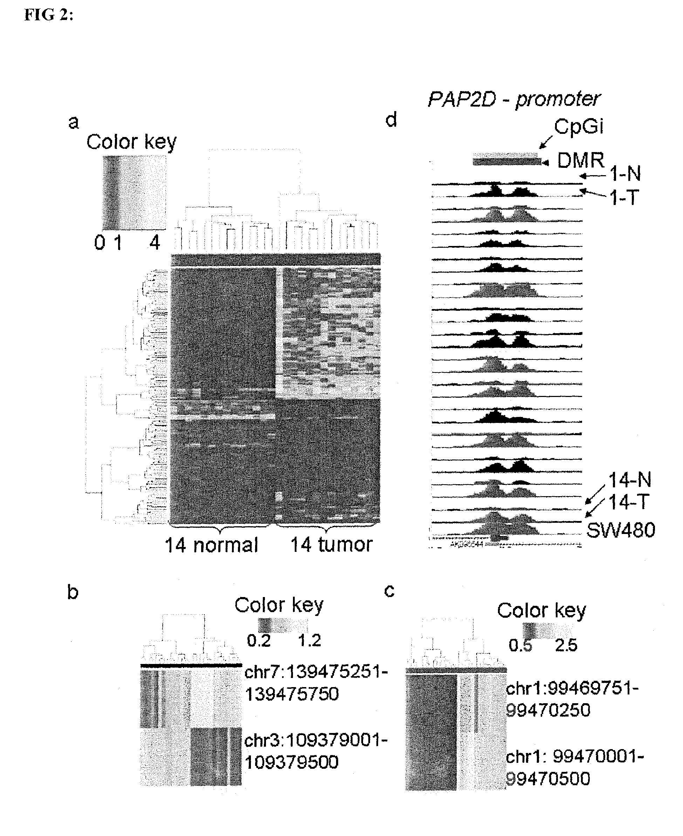

**Figure 2****: Biomarker analysis.** (a) Dendrogram of 158 cDMRs differentially methylated regions comparing tumor (red column labels) and normal tissue (blue column labels). DMRs were selected based on Wilcoxon's test between all samples. Only regions outside of CNAs and with a coefficient of variance below 0.5 were selected. Hierarchical clustering was performed with Canberra distance as pairwise distance measure and complete linkage as update rule using the R software (www.R-project.org). (b) An example of two DMRs sufficient for a correct discrimination of tumor and normal tissues. (c) An example of a single genomic region on chromosome 1 containing two overlapping DMRs that is related to clinical parameters. (d) Visualization of the region on chromosome 1 using the UCSC browser. RPM values are shown in wiggle format and show a consistent hypermethylation in the PAP2D promoter region. The maximal height for visualization was set to rpm=2 for all tracks. Panels show normal and tumor tissue for each patient as well as the SW480 cell line (bottom).

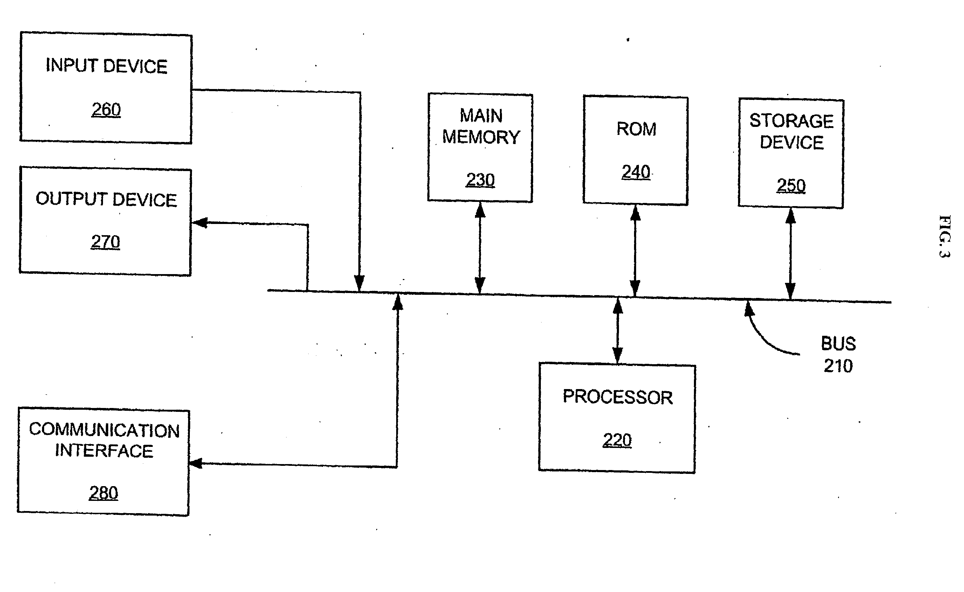

**FIG. 3** is an exemplary diagram of a computing device comprising a client and/or server according to an implementation consistent with the principles of the invention.
**FIG. 4** is a flowchart of exemplary processing of methylation status for biomarker(s) present in biological samples according to an implementation consistent with the principles of the present invention.

### EXAMPLES

### Experimental Procedure

**Tissue samples, DNA and RNA isolation.** The study has been approved by the Ethical Committee of the Medical University of Graz. For recent samples patients have given their written informed consent. For samples older than 15 years no informed consent was available, therefore all samples and medical data used in this study have been irreversibly anonymized.

Human tissue obtained during surgery was snap-frozen in liquid nitrogen. Cryosections (3 µm thick) were prepared and stained with haematoxylin and eosin to evaluate tumor cell content. Dissections were performed under the microscope to achieve a tumor cell content of >80%. DNA isolation was performed using the QIAamp DNA Mini Kit (Qiagen, Hilden, Germany), according to the manufacturer's instructions. DNA from the SW480 cell line was isolated using phenol/chloroform extraction followed by ethanol precipitation. Concentrations were measured on a Nanodrop and quality was assessed on an agarose gel. 10 µg of DNA was treated with 1 µl RNAse A (10µg/µl) for lh at 37°C prior to fragmentation. Microsatellite stabilities were determined following Promega's MSI Analysis System Protocol.

CpG island methylator phenotype (CIMP) was determined by assessing the MeDIP methylation values of the marker regions described in Issa and Weisenberger et al. (Issa, J.P. CpG island methylator phenotype in cancer. Nat Rev Cancer 4, 988-993 (2004); Weisenberger, D.J. et al. CpG island methylator phenotype underlies sporadic microsatellite instability and is tightly associated with BRAF mutation in colorectal cancer. Nat Genet 38, 787-793 (2006)). A tumor was classified as CIMP positive if at least 3 marker-regions of the classical marker set1 displayed a MeDIP-rpm value >0.26 which corresponds to the 0.99 quantile of the non-enriched input sequence.

**Library preparation and methylated DNA immunoprecipitation (MeDIP)**. Genomic DNA of the colon cancer patients was sonicated as described in Parkhomchouk et al. (Parkhomchuk, D. et al. Transcriptome analysis by strand-specific sequencing of complementary DNA. Nucleic Acids Res 37, e123 (2009)) to a size range of 100-400 bp and purified using Qiagen's AllPrep protocol (Qiagen). Then, 5 µg of fragmented DNA was subjected to single end library preparations using the genomic DNA sample prep kit (#FC-102-1002, Illumina, San Diego, USA) according to the manufacturer's instructions with modifications: End repair was performed in 317 µl total volume with 0.25 mM dNTPs Mix, 0.1 U T4 DNA Polymerase, 0.03 u Polymerase I, Klenow DNA Polymerase I (large fragment) and 0.3 U T4 DNA Polynukleotide Kinase. For A-tailing a total volume of 88 µl in the presence of 0.2 mM dATP and 0.5 u Klenow Fragment (3->5'exo-) was used. Adapters were ligated in a total volume of 98 µl using 29 µl of 'Adapter oligo mix' and two times increased amounts of ligase. Subsequently, the libraries were used for methylated DNA immunoprecipitation (see below). Libraries were amplified after MeDIP and prior to size selection in a total volume of 30 µl using 20 % of the immunoprecipitated DNA or 40 ng of non-immunoprecipitated library (input) for 6 PCR-cycles. Amplified libraries were run on a 2 % agarose gel and fragments of 150-400 bp were excised (corresponding to insert sizes of 80-330 bp) and purified using the Quiaquick Gel Extraktion Kit (Qiagen). Size-selected libraries were quantified using the QuantIt dsHS Assay Kit on a Qubit fluorometer (Invitrogen, Darmstadt, Germany).

MeDIP was adapted from a previously published protocol (Weber et al., 2005). In brief, 10 µl of monoclonal antibody against 5-methylcytidine (#BI-MECY, Eurogentec, Cologne, Germany) were incubated over night with 40 µl Dynabeads M-280 sheep anti-mouse IgG (Invitrogen) in 500 µl 0.5% BSA/PBS, washed two times with 0.5% BSA/PBS and one time with IP-buffer (10 mM sodium phosphate (pH7.0), 140 mM NaCl, 0.25 % Triton X100). Prior to immunoprecipitation, the sequencing libraries were denatured for 1 min at 95°C. Subsequently, 4 µg library was immunoprecipitated for 4 h at 4 °C using a 5-methylcytidine antibody coupled to Dynabeads in a total volume of 230 µl IP-buffer. After immunoprecipitation, the beads were washed three times with 700 µl IP-buffer and then treated with 50 mM Tris-HCl, pH 8.0; 10 mM EDTA, 1% SDS for 15 min at 65 °C. The supernatant containing the methylated DNA (200 µl) was diluted with 200 µl 10 mM Tris pH 8,0, 1 mM EDTA, treated with proteinase K (0.2 µg/µl) for 2 h at 55°C, followed by phenol-chloroform-extraction and ethanol precipitation. The DNA was resuspended in 20 µl 10 mM Tris pH 8.5.

**Validation of the MeDIP-enrichment by quantitative PCR**. The successful enrichment of methylated DNA was controlled by quantitative PCR. The PCR reactions were carried out in 10µl volume in 384 well plates on a 7900 Fast Real-Time PCR system using SYBR Green PCR master mix (Applied Biosystems, Darmstadt, Germany). Relative enrichment was calculated by the ratios of the signals in the immunoprecipitated DNA versus input DNA for a methylated positive and an unmethylated negative control region. Enrichment factors of approximately 50fold were used as parameter for successful enrichment. Primer sequences for methylated and unmethylated control regions were kindly provided by Dr. Vardham Rakyan (Barts and The London School of Medicine and Dentistry) and Prof. Dr. S. Beck, (UCL, Cancer Institute, London) (methylated: #4994; unmethylated: #8804)

**Preparation of RNA-seq libraries.** 2 µg of total RNA were depleted for ribosomal RNA using the RiboMinus Eukaryote Kit for RNA-seq (Invitrogen) following the manufacturer's instructions. The RiboMinus depleted RNA was then used for the generation of RNA-seq libraries using a strand-specific protocol as described previously (Parkhomchouk et al., 2009).

**Next generation sequencing.** After library quantification at a Qubit (Invitrogen) a 10 nM stock solution of the amplified library was created. Then, 12pmol of the stock solution were loaded onto the channels of a 1.4 mm flow cell and cluster amplification was performed. Sequencing-by-synthesis was performed on an Illumina Genome Analyser (GAIIx). All MeDIP and input samples were subjected to 36 nt single read sequencing. The raw data processing was done with the Illumina 1.5 and 1.6 pipeline.

For each of the 29 MeDIP-samples approximately 16 to 32 million uniquely aligned single end reads were generated with a total of over 22 Gb of MeDIP- and 11 Gb of input sequences. On average 69% of the generated reads for the input and 45% of the generated MeDIP-seq reads were uniquely aligned suggesting that approximately 24% of the generated reads (methylated DNA fragments) were located within repetitive sequences.

**Bisulfite treatment and PCR.** Bisulfite treatment was performed using standard protocols. Briefly, 500 ng genomic DNA was treated with 2 M sodium bisulfite and 0.6 M NaOH. Two thermo spikes of 99 °C for 5 min were introduced followed by two incubation steps of 1.5 h at 50 °C. Purification was achieved by loading, desulfonation and washing on a microcon YM-50 column (Millipore, Schwalbach, Germany). Bisulfite DNA was eluted in 50 µl 1xTE. PCRs for validation of MeDIP-seq data were performed in 30 µl reaction volume in presence of 1 x reaction buffer (10 mM Tris-HCL (pH 8.6), 50 mM KCl, 1.5 mM MgC12), 0.06 mM of each dNTP, 200 nM each, forward and reverse primer, 1.25 U HotStart-IT DNA polymerase (USB, Staufen, Germany) and 2 µl template. Finally, 5 µl of the PCR reaction products were differentiated on a 1.5% agarose gel.

**SIRPH analyses.** The methylation indices at particular CpGs in MeDIP enriched regions were determined using single-nucleotide primer extension (SNuPE) assays in combination with ion pair reverse phase high performance liquid chromatography (IP RP HPLC) separation techniques (SIRPH) (see El-Maarri, O. SIRPH analysis: SNuPE with IP-RP-HPLC for quantitative measurements of DNA methylation at specific CpG sites. Methods Mol Biol 287, 195-205 (2004)). In brief, 5 µl of each PCR product was purified using an ExonucleaseI/SAP mix (1U each, USB, Cleveland, USA) for 30 min at 37° C followed by a 15 min inactivation step at 80° C. Then, 14 µl primer extension mastermix (50 mM Tris-HCL, pH9.5, 2.5 mM MgC12, 0.05 mM ddCTP, 0.05 mM ddTTP, 3.6 µM of each SNuPE primer) was added and SNuPE reactions were performed. Obtained unpurified products were loaded on a DNASepTM (Transgenomic, Omaha, USA) column and separated in a primer-specific acetonitril gradient on the WAVETM system (Transgenomic). Methylation indices (MI) were obtained by measuring the peak heights (h) and calculating the ratio h(C)/[h(C)+h(T)]. To confirm the methylation assignment across the DMRs the second CpG position in most amplicons was analyzed in addition. For the SIRPH analyses 17 regions were selected and the analyses were performed for three patients and the colon cancer cell line SW480. Median Pearson's correlation values of 0.941 between the rms values (see below) of the MeDIP-seq and the methylation indices of the SIRPH results were achieved.

**Bisulfite pyrosequencing.** 454 GS-FLX: Amplicons were generated using region-specific primers with the recommended adaptors at their 5'-end. PCRs were performed in 30 µl reaction volumes in presence of 10 mM Tris-HCL (pH 8.6), 50 mM KCl, 1.5 mM MgCl2, 0.06 mM of each dNTP, 200 nM each, forward and reverse primer, 1.25 U HotStart-IT DNA polymerase (USB, Staufen, Germany) and 2 µl template. For the amplicons BMP1 and 'T' the usage of 1.5 U HotStarTaq and Q-Solution (Qiagen, Hilden, Germany) was necessary instead of HotStart-IT to obtain specific PCR products. Specific primer sequences and PCR protocols are provided in Supplementary Table 9. Amplicons were purified, measured using the Qubit Fluorometer (Invitrogen) and pooled. After emPCR, DNA containing beads were recovered, enriched and loaded onto a XLR70 Titanium PicoTiterPlate according to the manufacturer's protocols. Methylation level and pattern was assessed using multiple sequence alignment with an extended and improved version of BiQ Analyzer6. For the bisulfite pyrosequencing 25 regions in two patients were investigated and Pearson's correlations for the log2 ratios of tumor vs. normal of 0.842 (0.840) and 0.849 (0.859) for the rpm (rms) and bisulfite values were obtained.

**Alignment and pre-processing of sequencing reads.** Single end sequencing reads (36 bp) generated from MeDIP-seq experiments and input samples were aligned to the human genome (UCSC hg19) using Bowtie (version 0.12.5 parameter set -q -n 2 -k 5 --best-maxbts 10000 -m 1) allowing up to 2 nucleotide mismatches to the reference genome per seed and returning only uniquely mapped reads. Replicate sequencing reads (i.e. reads with exactly the same starting position) were counted only once.

The analysis of the MeDIP-seq data was performed with the MEDIPS package described in Chavez, L. et al. Computational analysis of genome-wide DNA methylation during the differentiation of human embryonic stem cells along the endodermal lineage. Genome Res 20, 1441-1450 (2010). For each MeDIP-seq and its corresponding input sample, the aligned reads were extended to 300 nt in the sequencing direction. The short read coverage of the extended reads was calculated at genome wide 50 bp bins. Subsequently, the final short read count at each genomic bin is transformed into reads per million format (rpm = number of reads in the bin / number of uniquely aligned reads x 1000000) (see Mortazavi, A., Williams, B.A., McCue, K., Schaeffer, L. & Wold, B. Mapping and quantifying mammalian transcriptomes by RNA-Seq. Nat Methods 5, 621-628 (2008)). Saturation analyses were performed to estimate the required read depth.

**Identification of cancer differentially methylated regions (cDMRs) between tumor and normal samples.** Mean rpm values were calculated for genome-wide 500 bp windows overlapping by 250 bp using MEDIPS. Subsequently, for each 500 bp window, we applied a Wilcoxon"s test in order to assess significance of methylation differences between the 14 controls (normal mucosa samples) and the 14 tumor samples. P-values were adjusted using the method of Benjamini and Yekutieli (2001) after exclusion of the mitochondrial and the sex chromosomes. Differentially methylated regions (cDMRs) were identified by filtering for 500 bp windows associated with adjusted p-values < 0.05. Overlapping significant 500 bp windows were merged if their ratios indicated the same hyper- or hypomethylated status. In order to assure that signals within DMRs are above background noise, a ratio of MeDIP versus input rpm-values > 1.5 was required. Here, the MeDIP/input ratio is calculated either for the tumor sample (hypermethylation) or for the normal sample (hypomethylation). In addition, only cDMRs outside of copy number alterations (CNAs) were considered (i.e. none of the patients in our sample set displayed a copy number alteration). Finally, the resulting significant CNA-free DMRs were selected with respect to a minimal p-value and coefficient of variance.

In order to visualize the performance of epigenetic biomarkers for discriminating between tumor and normal samples we performed hierarchical cluster analysis using Canberra distance as pairwise distance measure and complete linkage as update rule using the R software package.

Furthermore, plausible associations between the selected group of 158 cDMRs and clinico-pathological characteristics were evaluated using one independent generalized linear model with a quassi-poisson link for each clinical characteristic under consideration (CIMP status, grade, localization, histology, lymphatic node as absent or present, pT, sex, age as younger than or equal to 55 or older or equal than 70). In all the models the response was the rpm values for each tumor. Only conditions with more than one patient were assessed.; p-values below 0.05 were considered as significant and in Table 2 the clinical characteristics significant for more than 5% of the tested cDMRs (>8 single significant cDMRs) were reported.

**Table 2: Most significant cDMRs in CNA-free regions with impact on clinical features (lymph node status, CIMP status and histology).**

| **Chr** | **Start** | **End** | **HUGO gene name** | **Repeat class** | **Ratio T vs N** | **Lymph node pvalue** | **CIMP pvalue** | **Histology pvalue** |
|---|---|---|---|---|---|---|---|---|
| chr1 | 77334501 | 77335000 | ST6GALNAC5 | | 3,8 | 0,041 | 0,094 | 0,109 |
| chr1 | 99469501 | 99470250 | RP11-254021.1; RP5-896L10.1 | Simple repeat | 3,7 | 0,379 | 0,025 | 0,061 |
| chr1 | 99470501 | 99471000 | RP11-254021.1; RP5-896L10.1 | Low complexity | 4,8 | 0,193 | 0,047 | 0,123 |
| chr1 | 158151251 | 158151750 | CD1D | | 4,0 | 0,279 | 0,011 | 0,255 |
| chr1 | 170630001 | 170630500 | | | 3,9 | 0,104 | 0,033 | 0,107 |
| chr1 | 177133501 | 177134000 | ASTN1 | Low complexity | 7,6 | 0,139 | 0,043 | 0,086 |
| chr1 | 181452501 | 181453000 | CACNA1E | Simple repeat | 3,1 | 0,265 | 0,037 | 0,076 |
| chr1 | 181638501 | 181639000 | CACNA1E | LINE | 0,4 | 0,047 | 0,767 | 0,304 |
| chr1 | 217313001 | 217313750 | | Low complexity | 3,9 | 0,012 | 0,695 | 0,364 |
| chr2 | 7101001 | 7101500 | AC017076.1; AC013460.1; RNF144A | Simple repeat | 3,0 | 0,302 | 0,016 | 0,676 |
| chr2 | 40679501 | 40680000 | SLC8A1 | | 2,7 | 0,721 | 0,042 | 0,588 |
| chr2 | 55062251 | 55062750 | EML6 | LINE | 0,6 | 0,034 | 0,696 | 0,236 |
| chr2 | 66653751 | 66654250 | AC092669.5 | | 3,1 | 0,374 | 0,040 | 0,255 |
| chr2 | 115919751 | 115920750 | DPP10 | Simple repeat | 7,6 | 0,232 | 0,007 | 0,075 |
| chr3 | 149374751 | 149375250 | WWTR1; RP11-255N4.2 | | 3,2 | 0,591 | 0,047 | 0,089 |
| chr3 | 192128001 | 192128500 | FGF12 | Low complexity | 4,6 | 0,033 | 0,411 | 0,768 |
| chr4 | 20254751 | 20255500 | SLIT2 | | 5,7 | 0,032 | 0,362 | 0,361 |
| chr4 | 188666001 | 188666500 | | LINE | 0,4 | 0,009 | 0,418 | 0,821 |
| chr5 | 61041001 | 61041500 | CTD-2170G1.1 | LTR | 0,5 | 0,021 | 0,568 | 0,853 |
| chr5 | 173602501 | 173603000 | | LTR | 0,5 | 0,434 | 0,078 | 0,031 |
| chr6 | 36808251 | 36809000 | | | 3,4 | 0,000 | 0,494 | 0,675 |
| chr6 | 137322751 | 137323250 | IL20RA | | 0,4 | 0,008 | 0,737 | 0,796 |
| chr6 | 151561001 | 151561500 | AKAP12 | | 3,5 | 0,017 | 0,125 | 0,407 |
| chr7 | 79083751 | 79084250 | AC004945.2 | | 3,5 | 0,008 | 0,365 | 0,497 |
| chr7 | 98466751 | 98467500 | TMEM130 | | 7,4 | 0,539 | 0,024 | 0,312 |
| chr10 | 3805001 | 3805500 | RP11-184A2.3 | | 0,5 | 0,046 | 0,537 | 0,557 |
| chr10 | 7454751 | 7455500 | | | 6,0 | 0,369 | 0,029 | 0,059 |
| chr10 | 57389751 | 57390500 | | | 4,8 | 0,008 | 0,189 | 0,047 |
| chr12 | 3602251 | 3603000 | PRMT8 | | 9,2 | 0,476 | 0,014 | 0,006 |
| chr12 | 5019001 | 5019500 | KCNA1 | | 13,5 | 0,043 | 0,248 | 0,184 |
| chr12 | 5019751 | 5020750 | KCNA1 | | 6,9 | 0,044 | 0,014 | 0,012 |
| chr12 | 72667251 | 72667750 | AC087886.1; TRHDE | | 6,8 | 0,021 | 0,254 | 0,159 |
| chr12 | 95942751 | 95943250 | USP44 | | 6,1 | 0,361 | 0,002 | 0,016 |
| chr12 | 101916501 | 101917250 | | DNA;SINE | 0,4 | 0,211 | 0,530 | 0,150 |
| chr16 | 55364501 | 55365000 | IRX6 | | 3,7 | 0,003 | 0,241 | 0,258 |
| chr17 | 32908001 | 32908500 | TMEM132E | Low complexity | 7,4 | 0,067 | 0,047 | 0,515 |
| chr19 | 15090751 | 15091250 | | SINE | 3,7 | 0,244 | 0,028 | 0,008 |
| chr19 | 56904751 | 56905250 | ZNF582;AC00 6116.1 | | 7,6 | 0,570 | 0,153 | 0,049 |
| chr19 | 58125751 | 58126250 | | LINE | 3,9 | 0,112 | 0,021 | 0,004 |

**Annotation of the cDMRs.** Each DMR was annotated using ENSEMBL v589. Annotation included gene structures, transcripts, promoter regions (defined as -2 kb downstream and +500 bp upstream of the transcription start site), exons and introns. Furthermore, CpG islands were identified according to the criteria of Takai and Jones (Takai, D. & Jones, P.A. Comprehensive analysis of CpG islands in human chromosomes 21 and 22. Proc Natl Acad Sci U S A 99, 3740-3745 (2002)) and the UCSC annotation. CpG island shores were defined as 1 kb regions upstream or downstream of a CpG island. DMRs were annotated with repetitive regions using the repeat masker table provided by UCSC. CDMRs overlapping conserved elements were identified using the table browser function of the UCSC genome browser (hg19) and the phastConsElements46wayPrimates track (The Genome Sequencing Consortium, 2001; Fujita, P.A. et al. The UCSC Genome Browser database: update 2011. Nucleic Acids Res 39, D876-882 (2011); Karolchik, D. et al. The UCSC Table Browser data retrieval tool. Nucleic Acids Res 32, D493-496 (2004); Kent, W.J. et al. The human genome browser at UCSC. Genome Res 12, 996-1006 (2002)). For a comparison with colorectal cancer specific cDMRs identified previously by a restriction enzyme based approach and array hybridization, the cDMRs presented by Irizarry et al. (Irizarry, R.A. et al. The human colon cancer methylome shows similar hypo- and hypermethylation at conserved tissue-specific CpG island shores. Nat Genet 41, 178-186 (2009)) were converted from the hg18 to the hg19 version using the Batch Coordinate Conversion (liftOver) tool provided by UCSC. The resulting genomic positions were prolonged by 500 bp in each direction and an intersection with the cDMRs identified in this study was determined.

**CNA analysis.** Copy number alterations were detected using CNV-seq by calculating log2-ratios of read counts of the input sequences in tumor and normal tissue per patient in overlapping 25 kb windows along the genome15. The windows overlap by half of their total size (i.e. 12.5 kb). We run CNV-seq with the parameter set: --window-size 25000-log2-threshold 0.6 --p-value 0.005 --minimum-windows-required 1 --genome-size 3095693983 --global-normalization --annotate. P-values were computed based on a Gaussian distribution of the log2-ratios. Subsequently, CNV-seq combined overlapping windows that exceeded both the log2-ratio and p-value thresholds (0.6 and 0.005) and recalculated p-values and log2-ratios for these CNA regions. The detected CNA regions were annotated with exons using BioMart/ ENSEMBL v58.

**RNA-seq analysis.** 36mer RNA-seq reads were aligned to the human genome using Bowtie (version 0.12.5 - parameter set: -n 2 -1 36 -y --chunkmbs 256 --best --strata -k 1 -m 1) against the genomic reference UCSC hg19. Subsequently, reads that did not map to the genome were aligned to the cDNA reference ENSEMBL v58 in order to map reads spanning exon junctions. Then, uniquely mapped reads aligning to the sense strand of a gene were counted. Differential expression was calculated using the R/BioConductor edgeR package16. Genes were assigned as differentially expressed if the absolute log2 fold-change values were greater than 0.5.

**Correlation of gene expression, copy number and methylation.** A total set of 49,646 genes from ENSEMBL v58 was evaluated in order to determine the interdependence of expression levels, copy number and methylation status.

The methylation status was determined in the promoter region of the genes (defined as 1 kb upstream and 500 bp downstream of the TSS). Here, Wilcoxon's test was performed with the MeDIP-seq data of the individual patient comparing tumor versus normal tissue using 10 adjacent 50bp bins for each 500 bp window in the promoter region. Promoter regions with at least two consistent DMRs with significant corrected p-values <0.1 were considered as hypo- or hypermethylated respectively.

An association analysis was conducted using a qualitative measure for the copy number status (deletion, CNA-free and amplification) and for the methylated status (hypo-, hypermethylated, non-consistent). Expression was considered either quantitatively using the whole set of log2 expression fold-changes (Fig. 1f), or qualitatively counting only differentially expressed genes (Fig. 1e). For two-sided comparisons (expression versus CNA and CNA versus methylation), quantitative values for the fold-changes were used (Fig. 1d,f). In order to assess associations between copy number or methylation status and gene expression a Kruskal Wallis test was applied to compare the conditions simultaneously and a Wilcoxon test was applied to perform pairwise comparisons. In order to assess associations between methylation status and gene expression given a certain CNA status we evaluated 2x2 contingency tables with an exact Fisher test (Fig. 1 e).

### Results

In order to gain a clearer view of the relationships between cytosine methylation, CNAs and the transcriptome we generated genome-wide maps with high-throughput sequencing (HTS) technologies in combination with methylated cytosine specific immunocapturing (MeDIP-seq) for the analyses of 14 heterogeneous colorectal cancers with matched-pair tumor and normal tissues, as well as for the colorectal cancer cell line SW480 as a reference (Table 3). Pairwise Pearson's correlation coefficients indicate on average a greater homogeneity of normal mucosa (0.84 to 0.94), compared to tumor tissue (0.76 to 0.90).

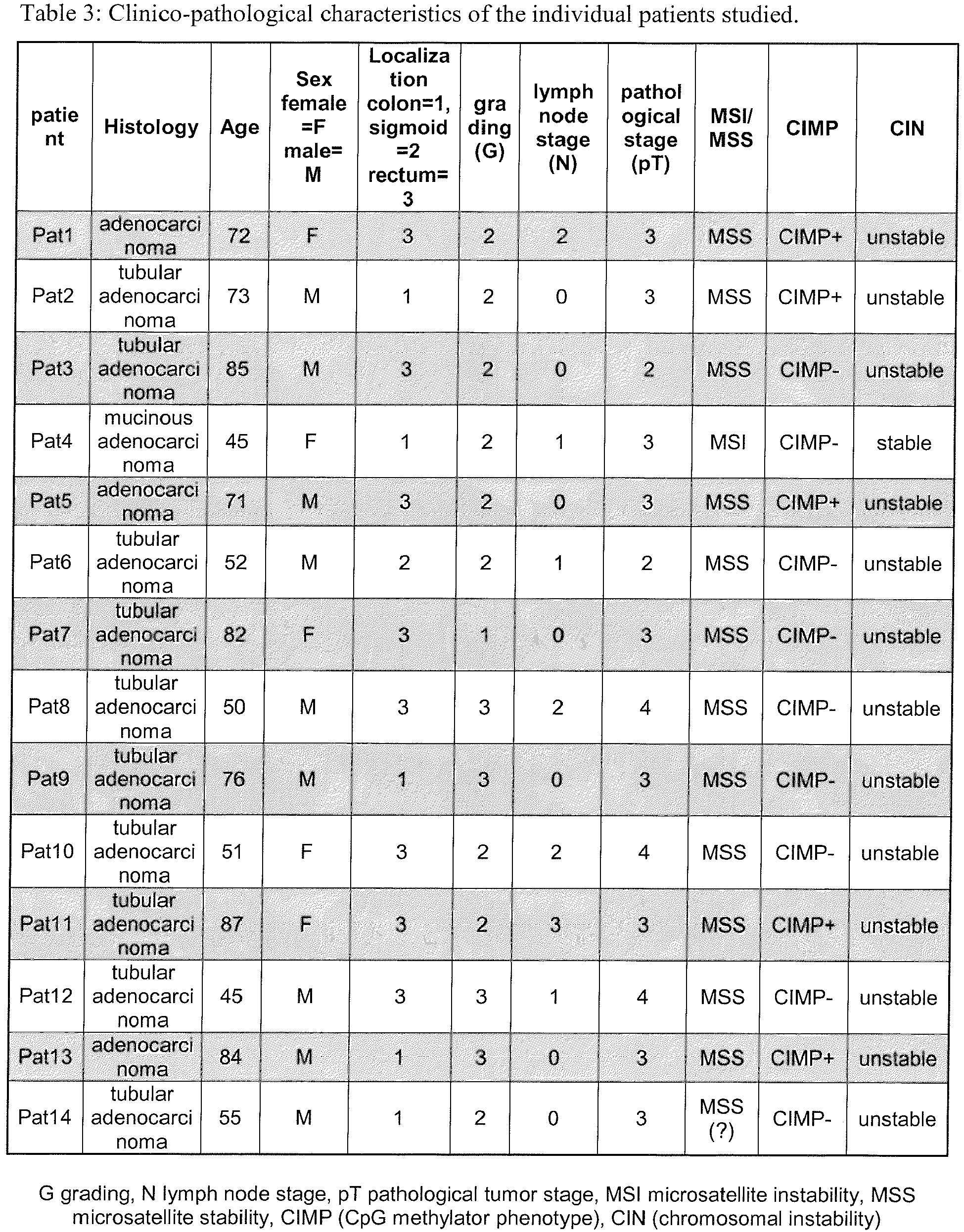

Using a robust non-parametric statistical test in a sliding window approach we identified a total of 7,912 cancer differentially methylated regions (cDMRs), corresponding to 4,381 merged cDMRs (1,673 tumor hyper-, and 2,708 tumor hypo- methylations). The majority (81%) of the tumor hypermethylation marks were located within CpG islands (1,358 cDMRs) and approximately 50% resided in promoters (839 cDMRs). In contrast, most tumor-specific hypomethylations were found in repetitive regions. Within our data set, we observed hypermethylations in low complexity regions and simple repeats, whereas most transposable elements, such as LINE, SINE and LTRs, were demethylated in tumor.

We were able to confirm several cDMRs known to be differentially methylated in cancer and which are described as potential biomarkers like EYA2, UCHL1, LRRC3B, HACE1, BAGE, MLH1, TMEFF2, NGFR, BMP3, ALX4, APC, DAPK, MGMT or SEPT9. However, based on the methylation values a complete discrimination between normal and tumor tissue was not possible or the markers are located within CNA containing regions (UCHL1 and LRRC3B).

To assess the validity of the large number of previously unknown cDMRs found in our study, MeDIP-seq data were validated using two different bisulfite-based validation techniques: methylation-specific single-nucleotide primer extension (SNuPE) followed by HPLC separation (SIRPH), as well as bisulfite pyrosequencing. Both, SIRPH analyses and bisulfite pyrosequencing, strongly correlated with the MeDIP-seq findings (0.94 and 0.85, respectively) indicating a high level of agreement between these techniques.

Our data gives evidence for genome-wide correlations of somatic CNA and methylation patterns (Fig. 1a,b). Most CNAs were detected in a single, or a low number, of patients (Fig. 1c) and, thus, might bias the discovery of epigenetic biomarkers (Fig. 1d). In addition, CNAs are thought to be partly responsible for transcriptome dosage effects. Therefore, we quantified the expression levels of 49,646 genes with RNA-seq and correlated them with copy number and promoter methylation changes. Indeed, we found a positive correlation between CNA and gene expression (Fig. 1e,f). As cytosine methylation is largely thought to result in transcriptional repression either by interfering with transcription factor binding or by induction of a repressive chromatin structure, we were interested to see whether these effects could be observed on a genomic scale.

Most of the large-scale associations between epigenome and the transcriptome have been studied within normal tissues and the question remains if an aberrant methylation pattern in cancer results in a concomitant misregulation of gene expression. Taking into account promoter methylation and gene expression across the genome, our data gives no evidence per se to support the hypothesis that promoter methylation leads to downregulation of gene expression. However, since we did observe an association between CNAs and gene expression (Fig. 1f), we correlated methylation and expression in CNA-free and affected regions separately. In contrast to the global promoter methylation analyses here we were able to detect significant correlations between hypermethylation and gene silencing and of hypomethylation with an increase in gene expression. Fig. 1e shows that in CNA free regions there are 12% more up-regulated compared to down-regulated genes, associated with hypomethylated promoters, whereas this trend is reversed for genes with hypermethylated promoters, where we observed 6% more down-regulated genes compared to up-regulated genes. This significantly connects promoter hypermethylation with down-and promoter hypomethylation with up-regulation of gene expression (Fisher test P=0.006); an effect that cannot be observed without corrections for CNAs. It is not clear from these data if the alteration in the methylation pattern within CNA regions observed is due to differing immunoprecipitation yields arising from variation in DNA levels, or if it is a physiological response to compensate differential gene expression arising from copy number alterations. This mechanism might not occur in a linear manner and simple proportional normalizations might be problematic. Taken together, we conclude that copy number aberrations impair the correlation between transcript and DNA methylation levels in the respective regions.

In particular for the identification of biomarkers this conclusion plays an important role: Within our patient's cohort we find CNA-free regions to be consistently represented across many patients (Fig. 1c). Here we detected 1,483 cDMRs (out of the 7,912 significant cDMRs described earlier) free of CNAs for all of the patients including 158 highly statistically robust regions, highlighting them as extremely attractive options for biomarker development (significant p-value <0.00684 after correction for multiple testing and lowest coefficients of variance <0.5) (Fig. 2a). Of these regions, already two were able to accurately classify the patients' tissues (Fig. 2b). Finally, we correlated these DMRs with the clinical parameters of the patients and derived a potential biomarker subset associated with CIMP status, histological observation and lymph node status (Table 2). Strikingly, we find among this subset that even one single region on chromosome 1 (composed of two overlapping significant cDMRs), can successfully separate tumor from normal tissue (Fig 2c, d). This means, for classification two regions are required, while for diagnosis a single genomic region that is selected from the group of Table 1 is sufficient.

The performance of this biomarker, and others found in CNA-free regions of the tumor genome, outperforms that of recently suggested biomarkers, SEPT9 or ALX425. The variable performance of these biomarkers may be linked to their location within CNAs in two (four for ALX4) patients studied here. For other regions described in the literature such as BRAF, MLH1 or APC we do not find significant differential methylation over the patients (see above). Our findings challenge the efficacy of using these biomarkers as general diagnostics.

Taken together, our results of the genome-wide interplay between CNAs, methylome and transcriptome, have important implications on the use of cancer diagnostic assays. We propose here that clinical analysis of cDMRs in regions devoid of CNAs could eliminate variation, decrease failure rate, and thus improve the predictive power of such assays. These quality control steps will make it possible in the future to identify methylation marks as robust biomarkers for the diagnosis and the prediction of tumor progression and response.

## Claims

1. A method for diagnosis of colorectal cancer, comprising the steps of
a. analysing in a sample of a subject the DNA methylation status of at least one genomic region selected from the group of Table 1,
b. wherein, if the at least one genomic region is differentially methylated, the sample is designated as colorectal cancer positive.

2. The method according to claim 1, wherein the at least one genomic region is selected from the group of:
a. Genomic region number (GR NO.) 1 to genomic region number 30;
b. Genomic region number 1 to genomic region number 20;
c. Genomic region number 1 to genomic region number 10;
d. Genomic region number 1 to genomic region number 5;

3. The method according to claim 1, wherein the at least one genomic region is genomic region number 1.

4. The method according to any of the preceding claims, wherein the genomic region is located in a region that is free of copy number alterations (CNAs).

5. The method according to any of the preceding claims, wherein the methylation status of a further genomic region and/or a further biomarker is analysed.

6. The method according to any of the preceding claims, wherein analysing the methylation status of a genomic region means analysing the methylation status of at least one CpG position per genomic region.

7. The method according to any of the preceding claims, wherein the methylation status is analysed by non-methylation-specific PCR based methods, methylation-based methods or microarray-based methods.

8. The method according to claim 7, wherein the methylation status is analysed by Epityper and Methylight (qPCR) assays.

9. The method according to any of the preceding claims, wherein the methylation status is calculated as a ratio of the percentage of methylated DNA of the biomarker in the sample to the percentage of non-methylated DNA of the biomarker in the sample.

10. The method according to any of the preceding claims, wherein the measuring step is conducted by a computing device.

11. The method according to any of the preceding claims, wherein the correlating step is conducted by a computing device.

12. The method according to any of the preceding claims, further comprising outputting for presentation on a display associated with the computing device.

13. A nucleic acid molecule that hybridizes under stringent conditions in the vicinity of one of the genomic regions according to genomic region number 1 to genomic region number 64, wherein said vicinity is any position having a distance of up to 500 nt from the 3' or 5' end of said genomic region, wherein said vicinity includes the genomic region itself.

14. A nucleic acid according to claim 13, wherein the nucleic acid is 15 to 100 nt in length.

15. A nucleic acid according to claim 14, wherein the nucleic acid is a primer.

16. A nucleic acid according to claim 15, wherein the primer is specific for one of the genomic regions of claim 1.

17. A nucleic acid according to claim 13 or 14, wherein the nucleic acid is a probe.

18. A nucleic acid according to claim 17, wherein the probe is labelled.

19. A nucleic acid according to any of the claims 13 to 18, wherein the nucleic acid hybridizes under stringent conditions in said vicinity of one of the genomic regions after a bisulphite treatment of the genomic region.

20. Use of the nucleic acid of any of the claims 13 to 19 for the diagnosis of colorectal cancer.

21. A composition for the diagnosis of colorectal cancer comprising a nucleic acid according to claims 13 to 19.

22. A kit for the diagnosis of colorectal cancer comprising a nucleic acid according to claims 13 to 19.
